# FASCICULE DE BREVET EUROPEEN

(11) **EP 3 727 327 B1**
(45) Date de publication et mention de la délivrance du brevet: **15.05.2024**
(21) Numéro de dépôt: 18842436.0
(22) Date de dépôt: 20.12.2018
(51) Int. Cl.: A61K 8/9789, A61K 8/9761, A61Q 19/02, A61K 36/14, A61K 36/575, A61K 36/86

(54) **UTILISATION D'ABSOLUE DE MAGNOLIA POUR DEPIGMENTER LA PEAU**
VERWENDUNG VON MAGNOLIEN ABSOLUE IN DER KOSMETIK ZUM DEPIGMENTIEREN DER HAUT
USE OF MAGNOLIA ABSOLUTE IN COSMETICS FOR DEPIGMENTING THE SKIN

(30) Priorité: 21.12.2017 FR 1762760
(43) Date de publication de la demande: 28.10.2020
(73) Titulaire: Robertet S.A., 06130 Grasse (FR)
(72) Inventeur: PEGARD, Anthony, 06130 GRASSE (FR); KORMANN, Karine, 06560 VALBONNE (FR)
(74) Mandataire: Germain Maureau
(86) Numéro de dépôt international: PCT/FR2018/053455
(87) Numéro de publication internationale: WO 2019/122748

(56) Documents cités:
- CN-A- 105 832 583
- JP-A- 2000 159 622
- JP-A- 2002 003 336
- KR-B1- 101 179 589
- GIRARD L: "ETAL ACTUEL DE LA QUESTION DES CONCRETES ET DES ABSOLUES DE CONCRETES//PRESENT STATUS OF THE QUESTION OF CONCRETES AND CONCRETE ABSOLUTES", INDUSTRIES DE LA PARFUMERIE, XX, FR, vol. 2, 1 janvier 1947 (1947-01-01), pages 182-188,217, XP001246870, ISSN: 0367-9349
- JIN KYONG-SUK ET AL: "Juniperus chinensisand the functional compounds, cedrol and widdrol, ameliorate [alpha]-melanocyte stimulating hormone-induced melanin formation in B16F10 c", FOOD SCIENCE AND BIOTECHNOLOGY, THE KOREA SOC. OF FOOD SCIENCE AND TECHNOLOGY, HEIDELBERG, vol. 24, no. 2, 30 April 2015 (2015-04-30) , pages 611-618, XP035474121, ISSN: 1226-7708, DOI: 10.1007/S10068-015-0080-5 [retrieved on 2015-04-30]

## Description

La présente invention a pour objet l'utilisation cosmétique d'une absolue obtenue à partir de magnolia, éventuellement combinées à une absolue obtenue à partir de genièvre et/ou une absolue obtenue à partir de violette pour dépigmenter la peau.

La pigmentation de la peau résulte essentiellement de sa teneur en pigments mélaniques. La mélanine est produite dans les mélanocytes à la base de l'épiderme, puis transférée aux kératinocytes pour atteindre les couches supérieures de l'épiderme.

L'enzyme clé dans la première des deux étapes de la mélanogenèse est la tyrosinase (monophenol, L-dihydroxypheénylalanine (L-DOPA): oxygen oxydoréductase EC 1.14.18.1). Elle permet l'hydroxylation de la L-tyrosine en L-DOPA (produit o-diphénol) et lequel est ensuite oxydé en dopaquinone.

Bien que régulée par des facteurs intrinsèques (hormones, facteurs de transcription), et extrinsèques (UV), des dérèglements de la mélanogenèse peuvent survenir. Apparaissent alors des pertes de la pigmentation (leucodermies, albinisme occulocutané) ou au contraire des hyperpigmentations avec le chloasma (masque de grossesse) ou des lentigos (tâches de vieillesse).

Afin de pallier ces hyperpigmentations inesthétiques, de nombreuses études ont été menées en vue de trouver des composés éclaircissants : des agents mélano-toxiques, des inhibiteurs de transfert de mélanine aux kératinocytes, des inhibiteurs de tyrosinase. Cependant les composés trouvés se sont révélés néfastes : allergènes, mutagènes, cancérigènes et repro-toxiques. C'est par exemple le cas de l'acide kojique, inhibiteur de la tyrosinase par chélation du cuivre, utilisé comme agent dépigmentant. Controversé, son interdiction, déjà d'actualité au Japon, est en cours de discussion en Europe.

L'enzyme purifiée du champignon *Agaricus bisporus* est couramment utilisée mais peut parfois donner des résultats très différents par rapport à la tyrosinase humaine.

Dans le cadre de la recherche d'actifs cosmétiques permettant de dépigmenter la peau, il est donc intéressant d'identifier des produits permettant d'inhiber la mélanogénèse en inhibant la tyrosinase qui induit la synthèse de mélanine à partir de tyrosine, mais ne présentant pas les défauts énoncés précédemment.

L'utilisation cosmétique de certains extraits végétaux, notamment des extraits d'écorces de Magnolia, et plus particulièrement de *Magnolia sieboldii* ou de *Magnolia officinalis*, pour blanchir la peau est connue. Les extraits végétaux utilisés en cosmétique sont classiquement obtenus par extraction avec des solvants polaires, afin d'obtenir les molécules qui présentent une affinité avec l'eau. En particulier, les huiles essentielles s'obtiennent généralement par distillation à la vapeur d'eau.

KR101179589B1 décrit l'utilisation d'un extrait d'écorce de *Magnolia officinalis* pour dépigmenter la peau.

Jin Kyong-Suk et al. « Juniperus chinensis and the functional compounds, cedrol and widdrol, ameliorate α-melanocyte stimulating hormone-induced melanin formation in B16F10 cells », Food Science and Biotechnology, The Korea Soc. of Food Science and Technology, Heidelberg vol. 24, no. 2 (2015) pp611-618, enseigne que des extraits de genièvre inhibent la mélanogénèse.

JP2000 159622A décrit l'utilisation d'un extrait d'une plante du genre Viola pour inhiber la production de mélanine et pour blanchir la peau.

L'absolue est un extrait, en général obtenu à partir de matière végétale, largement utilisé dans l'industrie du parfum. Contrairement aux extraits végétaux classiquement utilisés en cosmétique, l'absolue est obtenue par transformation alcoolique d'une concrète, elle-même obtenue par extraction avec un solvant organique volatil. Les absolues peuvent contenir des molécules différentes de celles contenues dans les extraits végétaux cosmétiques usuels.Plus précisément, l'absolue est obtenue à partir de concrète, laquelle est généralement préparée par macération d'un végétal frais (fleurs, racines, feuilles, écorces, baies etc...) dans un solvant organique volatil hydrocarboné tel que l'hexane ou l'éther de pétrole, ou un mélange bisolvant tel que hexane/acétate d'éthyle, hexane/acétate d'isopropyle, hexane/alcool isopropylique, cyclohexane/acétate d'éthyle, cyclohexane/acétate d'isopropyle, ou cyclohexane/alcool isopropylique, ou des mélanges d'hydrocarbures linéaires ou cycliques en C6 avec l'acétate d'éthyle, l'acétate d'isopropyle ou l'alcool isopropylique ; dans du CO₂ à l'état supercritique ; ou dans un gaz liquéfié (ou un mélange de gaz liquéfiés) tel que le butane ou le 1,1,1,2-tétrafluoroéthane (HFC-134a). Le solvant est ensuite évaporé pour donner une pâte appelée concrète qui renferme des composés aromatiques, des cires et des composés huileux de la plante.

La concrète, qui contient des cires responsables de la turbidité de la solution et rendent celle-ci peu soluble dans la base du parfum, ne peut être utilisée en parfumerie alcoolique telle quelle mais peut être employée dans les savons.

L'élimination, au moins partielle, des cires est donc nécessaire. Cette élimination est obtenue par glaçage et filtration : après ajout d'alcool à la concrète, la solution alcoolique obtenue est homogénéisée sous forte agitation à une température d'environ 30°C à 60°C puis réfrigérée entre -5°C et -18°C de sorte que les cires ex concrètes peuvent être éliminées par précipitation et filtration. L'alcool est ensuite évaporé pour obtenir l'absolue.

Ainsi, l'utilisation d'absolues de magnolia, de genièvre ou de violette en parfumerie est connue. Néanmoins, l'utilisation de ces absolues en cosmétique, en particulier pour dépigmenter la peau, n'a jamais été rapportée ou suggérée.

Or, il a maintenant été trouvé de façon surprenante qu'une absolue obtenue à partir de magnolia, de genièvre ou de violette possède des propriétés inhibitrices de la tyrosinase particulièrement intéressantes, ce qui permet son utilisation cosmétique pour dépigmenter la peau (ou pour le blanchiment de la peau).

Ainsi, la présente invention a pour objet l'utilisation cosmétique d'une absolue obtenue à partir de magnolia, éventuellement combinée à une absolue obtenue à partir de genièvre et/ou une absolue obtenur à partir de violette pour dépigmenter la peau.

Dans le cadre de la présente invention on entend par « absolue » tout extrait de matière végétale susceptible d'être obtenu par tout procédé connu de l'homme du métier (voir par exemple : Girard L : « Etat actuel de la question des concrètes et des absolues de concrètes », Industries de la parfumerie, Vol. 2, 1 janvier 1947, pages 183-262), et notamment par le procédé suivant :
- préparation d'une concrète sous forme de pâte
   > par macération de la matière végétale fraîche dans un solvant organique volatil hydrocarboné tel que l'hexane ou l'éther de pétrole, ou un mélange de bisolvant tel que hexane/acétate d'éthyle, hexane/acétate d'isopropyle, hexane/alcool isopropylique, cyclohexane/acétate d'éthyle, cyclohexane/acétate d'isopropyle, ou cyclohexane/alcool isopropylique, ou des mélanges d'hydrocarbures linéaires ou cycliques en C6 avec l'acétate d'éthyle, l'acétate d'isopropyle ou l'alcool isopropylique; dans du CO₂ à l'état supercritique ; ou dans un gaz liquéfié (ou un mélange de gaz liquéfiés) tel que le butane ou le HFC134a ;
   > suivie de l'évaporation du solvant ou du mélange de solvant ;
- élimination, au moins partielle, des cires contenues dans la concrète ainsi obtenue pour obtenir l'absolue par
   > ajout d'alcool ;
   > homogénéisation de la solution ainsi obtenue sous forte agitation à une température d'environ 30°C à 60°C ;
   > réfrigération de la solution entre -5°C et -18°C et élimination des cires ex concrète par précipitation et filtration ;
   > et évaporation de l'alcool.

Le procédé de préparation de l'absolue peut être suivi si besoin d'une étape de purification de l'absolue par toute technique connue de l'homme du métier, notamment par distillations moléculaires ou en utilisant des résines échangeuses d'ions.

Les absolues peuvent être obtenues à partir de toutes les parties du végétal considéré, telles que par exemple le bourgeon, le cône, les aiguilles, la fleur, la racine, la sève, l'écorce, la baie, la tige ou la feuille.

De plus, dans le cadre de la présente invention :
- on entend par « magnolia » toute espèce de magnolia, et en particulier l'espèce *Magnolia officinalis* ;
- on entendu par « genièvre » toute espèce de genièvre, et en particulier l'espèce *Juniperus communis* ;
- on entend par « violette », toute espèce de violette, et en particulier l'espèce *Viola odorata* ; et
- on entend par « utilisation cosmétique pour dépigmenter la peau » toute utilisation non thérapeutique d'un produit en vue d'obtenir un éclaircissement et/ou une uniformisation de la teinte de la peau.

Enfin, dans le cadre de la présente invention, et sauf mention contraire, les proportions exprimées en % correspondent à des pourcentages massiques par rapport au poids total de l'entité considérée.

La présente invention a donc pour objet l'utilisation d'une absolueobtenue à partir de magnolia, et éventuellement une absolue obtenue à partir de genièvre et/ou une absolue obtenue à partir de violette pour dépigmenter la peau. De préférence, ladite (ou lesdites) absolue(s) possède(nt) tout ou partie des caractéristiques suivantes :
- l'absolue obtenue à partir de magnolia est obtenue à partir de l'écorce de magnolia ; de préférence encore à partir de l'écorce de *Magnolia officinalis* ;
- l'absolue obtenue à partir de genièvre est obtenue à partir de baie de genièvre, de préférence encore à partir de baies de *Juniperus communis* ; et/ou
- l'absolue obtenue à partir de violette est obtenue à partir de feuilles de violette, de préférence encore à partir de feuilles de *Viola odorata.*

De préférence également, les absolues utilisées dans le cadre de la présente invention ont été obtenues à partir d'une concrète elle-même obtenue par extraction à l'hexane.

Dans le cadre de la présente invention, l'absolue obtenue à partir de magnolia peut être utilisée seule ou en combinaison avec les absolues citées précédemment. Or, une synergie d'action a été observée lorsque l'on combine plusieurs des absolues citées précédemment. Ainsi, la présente invention a préférentiellement pour objet l'utilisation cosmétique de l'une des combinaisons d'absolues suivantes pour dépigmenter la peau :
- absolue obtenue à partir de magnolia telle que définie précédemment et absolue obtenue à partir genièvre telle que définie précédemment ;
- absolue obtenue à partir de magnolia telle que définie précédemment et absolue obtenue à partir de violette telle que définie précédemment ;ou
- absolue obtenue à partir de magnolia telle que définie précédemment, absolue obtenue à partir de genièvre telle que définie précédemment, et absolue obtenue à partir de violette.

Les absolues décrites dans le cadre de la présente invention sont donc utilisées en cosmétique pour leur effet dépigmentant. Pour ce faire, ces absolues peuvent être incorporées dans une composition cosmétique qui peut être formulée sous toute forme galénique appropriée à son administration, en particulier à son application sur la peau (par voie topique).

Les compositions cosmétiques comprenant l'extrait utilisé dans le cadre de la présente invention peuvent être formulées sous forme liquide, pâteuse, ou solide, et plus particulièrement sous forme d'onguents, de crèmes, de laits, de pommades, de poudres, de tampons imbibés (lingettes), de solutions, de gels, de sprays, de mousses, de suspensions ou de sticks. Elle peut également se présenter sous forme de suspensions de microsphères ou nanosphères, de vésicules lipidiques ou polymériques, ou de patches polymériques ou gélifiés permettant une libération contrôlée, mais également sous la forme de produits rincés tels que gels douche, produits de bain (sels, moussant, etc).

Ces compositions cosmétiques contiennent l'absolue (ou les absolues) utilisée(s) dans le cadre de la présente invention à des teneurs allant de 0,0001% à 10% en poids total de la composition, préférentiellement de 0,001% à 1% en poids total de la composition.

Pour la préparation de ces compositions, l'absolue utilisée dans le cadre de la présente invention est mélangée aux excipients classiquement utilisés dans le domaine cosmétique.

Les compositions comprenant l'absolue (ou les absolues) utilisée(s) dans le cadre de la présente invention peuvent prendre la forme de compositions parfumantes possédant une activité cosmétique, telle que par exemple les compositions de la gamme Actiscent^{®}.

Les compositions cosmétiques comprenant l'absolue (ou les absolues) utilisée(s) dans le cadre de la présente invention peuvent prendre la forme d'une crème dans laquelle ladite fraction est associée aux excipients couramment utilisés en cosmétologie.

Ces compositions peuvent également prendre la forme de gels dans les excipients appropriés tels que les esters de cellulose ou d'autres agents gélifiants, tels que le carbopol, le sepinov (polyacrylate) ou la gomme guar.

Ces compositions peuvent aussi prendre la forme d'une lotion ou d'une solution dans lesquelles l'extrait utilisé dans le cadre de la présente invention est sous forme encapsulée. Les microsphères peuvent par exemple être constituées de corps gras, d'agar et d'eau. L'extrait utilisé dans le cadre de la présente invention peut être incorporé dans des vecteurs de type liposomes, glycosphères, cyclodextrines, dans des chylomicrons, des macro-, micro-, nano-particules ainsi que les macro-, micro- et nanocapsules et aussi être absorbés sur des polymères organiques poudreux, les talcs, bentonites et autres supports minéraux.

Ces émulsions jouissent d'une bonne stabilité et peuvent être conservées pendant le temps nécessaire pour l'utilisation à des températures comprises entre 0 et 50°C sans qu'il y ait sédimentation des constituants ou séparation des phases.

Les compositions cosmétiques comprenant l'absolue (ou les absolues) utilisée(s) dans le cadre de la présente invention peuvent aussi contenir des additifs ou des adjuvants usuels en cosmétologies, comme par exemple des agents antimicrobiens ou des parfums mais aussi des lipides d'extraction ou de synthèse, des polymères gélifiants et viscosifiants, des tensio-actifs et des émulsifiants, des principes actifs hydro- ou liposolubles, des extraits de plantes, des extraits tissulaires, des extraits marins, des actifs de synthèse.

Les compositions cosmétiques comprenant l'absolue (ou les absolues) utilisée(s) dans le cadre de la présente invention peuvent aussi comprendre d'autres principes actifs complémentaires choisis pour leur action, par exemple pour l'effet amincissant, l'effet anti-cellulite, l'effet raffermissant, l'effet hydratant, l'effet anti-âge, l'activité antimicrobienne, l'activité anti-oxydante, l'activité anti-radicalaire, l'effet cicatrisant, l'effet tenseur, l'effet anti-ride, l'activité chélatante, l'activité complexante et séquestrante, l'effet apaisant, l'effet anti-cernes, l'effet anti-rougeurs, l'activité émolliente, l'effet démêlant capillaire, l'activité anti-pelliculaire, l'effet stimulant de la repousse du cheveu, l'effet inhibant la chute du cheveu, l'effet gainant capillaire, l'activité épilatoire, l'activité limitant la repousse du poil, l'activité participant au renouvellement cellulaire, l'activité modulant la réponse inflammatoire, l'activité participant au maintien de l'ovale du visage, mais également la protection solaire, l'activité anti-irritante, la nutrition cellulaire, la respiration cellulaire, les traitements anti-séborrhéiques, la tonicité cutanée, la protection du cheveu.

Lorsque les compositions cosmétiques comprenant l'extrait utilisé dans le cadre de la présente invention contiennent des principes actifs complémentaires, ceux-ci sont généralement présents dans la composition à une concentration suffisamment élevée pour qu'ils puissent exercer leur activité.

Les compositions cosmétiques comprenant l'absolue (ou les absolues) utilisée(s) dans le cadre de la présente invention sont de préférence utilisées quotidiennement et appliquées une ou plusieurs fois par jour. Elles peuvent être appliquées sur toutes zones nécessitant une diminution de la pigmentation, notamment sur le visage, le crâne, les jambes, les mains, le torse, les bras ou le dos.

Enfin, dans un autre aspect, la présente invention a également pour objet une méthode pour réduire la pigmentation de la peau comprenant la sélection d'une zone de peau sur laquelle la réduction de la pigmentation est recherchée, et l'application sur ladite zone d'au moins une absolue choisie parmi les absolues obtenues à partir de magnolia, de genièvre ou de violette telle que définie précédemment en quantité suffisante pour réduire ladite pigmentation.

La présente invention est illustrée de manière non limitative par les exemples suivants.

### Exemple 1 - Efficacité in vivo des compositions de l'invention

### Préparation des absolues Magnolia

Dans un réacteur de 20 litres, 3 kg d'écorces de Magnolia broyées sont extraits avec 5 volumes d'hexane à température ambiante et sous agitation mécanique pendant 1h30. Le tout est mis à reposer pendant 30 minutes puis filtré sur plissé. Les écorces épuisées sont à nouveau extraites avec 5 volumes d'hexane dans les mêmes conditions que décrites précédemment.

Les deux filtrats d'extraction sont réunis et concentrés sous vide au Rotavapor (40°C sous 30 mbar).

On obtient 186.3 g d'un liquide visqueux brun vert (concrète) soit un rendement de 6.2%.

Dans un réacteur de 4 litres muni d'une agitation mécanique, la concrète ainsi obtenue (186.3 g) est dissoute dans 6 volumes d'alcool dénaturé et agitée pendant 1h à 45°C.

Puis l'ensemble est mis à glacer au congélateur pendant 12 heures.

La solution est filtrée sur fritté muni d'un lit de Célite. On recueille ainsi un premier filtrat. (S1)

Les cires résiduelles retenues sur fritté sont lavées à nouveau avec 2 volumes d'alcool dénaturé froid. On recueille ainsi un deuxième filtrat (S2).

Les deux filtrats sont concentrés sous vide séparément au Rotavapor (50°C sous 30 mbar). Un test de solubilité est effectué sur les deux filtrats concentrés individuellement pour vérifier qu'ils ne présentent aucun insoluble quand ils sont mis en solution à 10 % dans de l'alcool.

Les deux filtrats concentrés sont alors mélangés et on obtient 114.3 g d'un liquide brun orangé soit un rendement de 61.3% (absolue Magnolia).

### Préparation des absolues Genièvre et Violette

Le procédé décrit ci-avant a également permis d'obtenir les absolues suivantes :
- « absolue Genièvre » correspondant à l'absolue obtenue à partir de baies de *Juniperus communis* ; et
- « absolue Violette » obtenue à partir de feuilles de *Viola odorata.*

### Protocole expérimental

Le protocole suivant a permis de déterminer la concentration inhibitrice médiane (Cl50) des différentes absolues, seules ou en combinaison. La Cl₅₀ est une mesure de l'efficacité d'un composé donné pour inhiber une fonction biologique ou biochimique spécifique. Ici le Cl 50 correspond à la concentration à laquelle le rapport de la vitesse maximale de transformation du substrat en produit pour la condition sur la Vₘₐₓ de la transformation pour le témoin est de 50%.

Des tests sur modèle *in vivo* sur cellules productrices de mélanine, les mélanocytes murins B16-F10, ont été nécessaires. La diminution de l'activité de tyrosinase cellulaire, associée à une réduction du taux de mélanine intracellulaire, tout en conservant la survie des cellules par des concentrations non-cytotoxiques, ont alors été recherchées. Pour cela, les cellules B16-F10 ont été ensemencées dans des plaques 24 puits, et incubées pendant 24h à 37°C avec plusieurs concentrations de différentes matières premières sélectionnées. L'acide kojique a également été utilisé comme produit de référence. Les cellules sont ensuite lysées, et centrifugées.

Le surnageant contient la tyrosinase que l'on dépose dans une plaque 96 puits et à laquelle on ajoute du substrat L-DOPA, puis lecture de l'absorbance à 490 nm toutes les 10 min pendant 1 heure. Le rapport de la vitesse maximale de la condition / vitesse maximale du contrôle négatif, donne le taux d'inhibition de la condition et permet d'estimer les Cl₅₀ de chaque matière première.

Le culot cellulaire quant à lui, est dissous par une solution de NaOH 1N et une incubation d'une heure à 70°C avant d'être déposé dans une plaque 96 puits passée au spectrophotomètre à 405 nm. Une comparaison avec l'absorbance d'une gamme étalon de mélanine standard permet ensuite de déterminer le taux de mélanine intracellulaire de chaque condition et de s'assurer de l'effet inhibiteur de la mélanogenèse par les différentes matières premières.

Un test de viabilité cellulaire au sel de tétrazolium MTT (bromure de 3-(4,5-dimethylthiazol-2-yl)-2,5-diphenyl tetrazolium) est effectué. Pour cela les cellules sont incubées à 37°C pendant 3h avec du milieu contenant 1mg/ml de MTT, avant de solubiliser les cristaux de formazan formés par du DMSO et lire l'absorbance à 570nm. Le taux de survie cellulaire est exprimé en pourcentage par rapport aux contrôles négatifs.

### Résultats

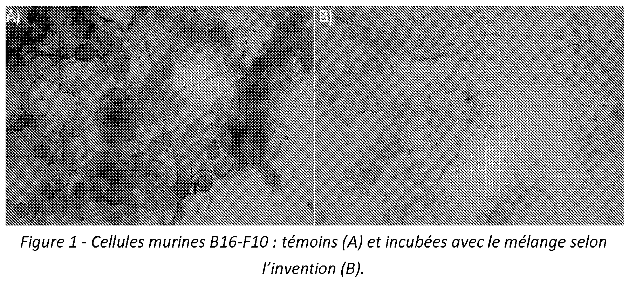

Les résultats obtenus sont rapportés dans les tableaux 1 et 2 suivants, ainsi que dans la figure 1 représentant des photographies de cellules murines B16-F10 témoins (A) et incubées avec le mélange Absolue Magnolia / Absolue Genièvre / Absolue Violette (1/1/1) (B).

**Tableau 1**

| **Tests sur mélanocytes B16, avec évaluation de l'activité de la tyrosinase intracellulaire** | | |
|---|---|---|
| **Ingrédient** | **CI50 effective (ppm)** | **CI50 théorique** |
| Absolue Magnolia | 18 | - |
| Absolue Genièvre (hors invention) | 9 | - |
| Absolue Violette (hors invention | 17 | - |
| Absolue Magnolia / Absolue Genièvre (1/1) | 7 | 12 |
| Absolue Magnolia / Absolue Violette (1/1) | 10 | 17 |
| Absolue Violette / Absolue Genièvre (1/1) (hors invention) | 4 | 13 |
| Absolue Magnolia / Absolue Genièvre / Absolue Violette (1/1/1) | 4,2 | 13 |
| Acide kojique | 225 | - |

**Tableau 2**

| **Tests sur mélanocytes B16, avec dosage de la mélanine intracellulaire.** | | |
|---|---|---|
| **Ingrédient** | **CI50 effective (ppm)** | **CI50 théorique** |
| Absolue Magnolia | 18,5 | - |
| Absolue Genièvre (hors invention) | 111 | - |
| Absolue Violette (hors invention) | 54 | - |
| Absolue Magnolia / Absolue Genièvre (1/1) | 20 | 32 |
| Absolue Magnolia / Absolue Violette (1/1) | 18 | 28 |
| Absolue Violette / Absolue Genièvre (1/1) (hors invention) | 52 | 73 |
| Absolue Magnolia / Absolue Genièvre / Absolue Violette (1/1/1) | 21 | 37 |
| Acide kojique | 214 | - |

### Conclusion

Les résultats obtenus confirment l'activité inhibitrice de la tyrosinase et de la synthèse de la mélanine intracellulaire de chacune des absolues testées, et donc leur efficacité cosmétique pour dépigmenter la peau.

En outre, ces résultats mettent en avant l'existence d'une synergie d'action inattendue entre les différentes absolues. Cette synergie est observée pour les combinaisons de deux ou trois absolues.

## Revendications

1. Utilisation cosmétique d'une absolue obtenue à partir de magnolia pour dépigmenter la peau.

2. Utilisation selon la revendication 1, **caractérisée en ce que** l'absolue obtenue à partir de magnolia est obtenue à partir de l'écorce de magnolia.

3. Utilisation selon la revendication 2, **caractérisée en ce que** l'absolue obtenue à partir de magnolia à partir de l'écorce de *Magnolia officinalis.*

4. Utilisation selon l'une des revendications 1 à 3, **caractérisée en ce que** l'absolue de magnolia est combinée à au moins l'une d'une absolue obtenue à partir de genièvre et d'une absolue obtenue à partir de violette.

5. Utilisation selon la revendication 4, **caractérisée en ce que** l'absolue obtenue à partir de genièvre est obtenue à partir de baie de genièvre.

6. Utilisation selon la revendication 5, **caractérisée en ce que** l'absolue obtenue à partir de à partir de genièvre est obtenue de baies de *Juniperus communis.*

7. Utilisation selon l'une des revendications 1 à 6, **caractérisée en ce que** l'absolue obtenue à partir de violette est obtenue à partir de feuilles de violette.

8. Utilisation selon la revendication 7, **caractérisée en ce que** l'absolue obtenue à partir de à partir violette est obtenue à partir de feuilles de *Viola odorata.*

9. Utilisation cosmétique d'une absolue obtenue à partir de magnolia telle que définie dans l'une des revendications 1 à 3 combinée à une absolue obtenue à partir de genièvre telle que définie dans l'une des revendications 4, 5 ou 6 pour dépigmenter la peau.

10. Utilisation cosmétique d'une absolue obtenue à partir de magnolia telle que définie dans l'une des revendications 1 à 3 combinée à une absolue obtenue à partir de violette telle que définie dans l'une des revendications 4, 7 ou 8 pour dépigmenter la peau.

11. Utilisation cosmétique d'une absolue obtenue à partir de genièvre telle que définie dans l'une des revendications 4, 5 ou 6 combinée à une absolue obtenue à partir de violette telle que définie dans l'une des revendications 4, 7 ou 8 pour dépigmenter la peau.

12. Utilisation cosmétique d'une absolue obtenue à partir de magnolia telle que définie dans l'une des revendications 1 à 3 combinée à une absolue obtenue à partir de genièvre telle que définie dans l'une des revendications 4, 5 ou 6 et à une absolue obtenue à partir de violette telle que définie dans l'une des revendications 4, 7 ou 8 pour dépigmenter la peau.

## Patentansprüche

1. Kosmetische Verwendung eines aus Magnolien gewonnenen Absolues zum Depigmentieren der Haut.

2. Verwendung nach Anspruch 1, **dadurch gekennzeichnet, dass** das aus Magnolien gewonnene Absolue aus der Magnolienrinde gewonnen wird.

3. Verwendung nach Anspruch 2, **dadurch gekennzeichnet, dass** das aus Magnolien gewonnene Absolue aus der Rinde von *Magnolia officinalis* gewonnen wird.

4. Verwendung nach einem der Ansprüche 1 bis 3, **dadurch gekennzeichnet, dass** das Magnolien-Absolue mit mindestens einem eines aus Wacholder gewonnenen Absolues und eines aus Veilchen gewonnenen Absolues kombiniert wird.

5. Verwendung nach Anspruch 4, **dadurch gekennzeichnet, dass** das aus Wacholder gewonnene Absolue aus Wacholderbeeren gewonnen wird.

6. Verwendung nach Anspruch 5, **dadurch gekennzeichnet, dass** das aus Wacholder gewonnene Absolue aus Beeren von *Juniperus communis* gewonnen wird.

7. Verwendung nach einem der Ansprüche 1 bis 6, **dadurch gekennzeichnet, dass** das aus Veilchen gewonnene Absolue aus Veilchenblättern gewonnen wird.

8. Verwendung nach Anspruch 7, **dadurch gekennzeichnet, dass** das aus Veilchen gewonnene Absolue aus Blättern von *Viola odorata* gewonnen wird.

9. Kosmetische Verwendung eines aus Magnolien gewonnenen Absolues, wie in einem der Ansprüche 1 bis 3 definiert, kombiniert mit einem aus Wacholder gewonnenen Absolue, wie in einem der Ansprüche 4, 5 oder 6 definiert, zum Depigmentieren der Haut.

10. Kosmetische Verwendung eines aus Magnolien gewonnenen Absolues, wie in einem der Ansprüche 1 bis 3 definiert, kombiniert mit einem aus Veilchen gewonnenen Absolue, wie in einem der Ansprüche 4, 7 oder 8 definiert, zum Depigmentieren der Haut.

11. Kosmetische Verwendung eines aus Wacholder gewonnenen Absolues, wie in einem der Ansprüche 4, 5 oder 6 definiert, kombiniert mit einem aus Veilchen gewonnenen Absolue, wie in einem der Ansprüche 4, 7 oder 8 definiert, zum Depigmentieren der Haut.

12. Kosmetische Verwendung eines aus Magnolien gewonnenen Absolues, wie in einem der Ansprüche 1 bis 3 definiert, kombiniert mit einem aus Wacholder gewonnenen Absolue, wie in einem der Ansprüche 4, 5 oder 6 definiert, und mit einem aus Veilchen gewonnenen Absolue, wie in einem der Ansprüche 4, 7 oder 8 definiert, zum Depigmentieren der Haut.

## Claims

1. A cosmetic use of an absolute obtained from magnolia to depigment the skin.

2. The use according to claim 1, **characterized in that** the absolute obtained from magnolia is obtained from magnolia bark.

3. The use according to claim 2, **characterized in that** the absolute obtained from magnolia from the *Magnolia officinalis* bark.

4. The use according to any of claims 1 to 3, **characterized in that** the magnolia absolute is combined with at least one of an absolute obtained from juniper and an absolute obtained from violet.

5. The use according to claim 4, **characterized in that** the absolute obtained from juniper is obtained from juniper berry.

6. The use according to claim 5, **characterized in that** the absolute obtained from juniper is obtained from *Juniperus communis* berries.

7. The use according to any of claims 1 to 6, **characterized in that** the absolute obtained from violet is obtained from violet leaves.

8. The use according to claim 7, **characterized in that** the absolute obtained from violet is obtained from *Viola odorata* leaves.

9. The cosmetic use of an absolute obtained from magnolia as defined in any of claims 1 to 3 combined with an absolute obtained from juniper as defined in any of claims 4, 5 or 6 to depigment the skin.

10. The cosmetic use of an absolute obtained from magnolia as defined in any of claims 1 to 3 combined with an absolute obtained from violet as defined in any of claims 4, 7 or 8 to depigment the skin.

11. The cosmetic use of an absolute obtained from juniper as defined in any of claims 4, 5 or 6 combined with an absolute obtained from violet as defined in any of claims 4, 7 or 8 to depigment the skin.

12. The cosmetic use of an absolute obtained from magnolia as defined in any of claims 1 to 3 combined with an absolute obtained from juniper as defined in any of claims 4, 5 or 6 and an absolute obtained from violet as defined in any of claims 4, 7 or 8 to depigment the skin.
